# EUROPEAN PATENT SPECIFICATION

(11) **EP 2 967 878 B1**
(45) Date of publication and mention of the grant of the patent: **26.02.2020**
(21) Application number: 14765408.1
(22) Date of filing: 17.03.2014
(51) Int. Cl.: A61F 2/30, A61F 2/48, A61F 2/32, A61F 2/42, A61F 2/38, A61F 2/40, A61B 17/17, A61B 34/32, A61B 34/30, A61B 17/16, A61B 17/56, A61B 34/10, A61B 90/00

(54) **SYSTEM FOR CREATING UNIQUE PATTERNS IN BONE FOR CARTILAGE REPLACEMENT**
SYSTEM ZUR ERSTELLUNG EINDEUTIGER MUSTER IN KNOCHEN FÜR KNORPELERSATZ
SYSTÈME DE CRÉATION DE CAVITÉS OSSEUSES POUR GUÉRISON OSSEUSE

(30) Priority: 15.03.2013 US 201361787616 P
(43) Date of publication of application: 20.01.2016
(73) Proprietor: Think Surgical, Inc., Fremont, CA 94539 (US)
(72) Inventor: NETRAVALI, Nathan A., Palo Alto, California 94306 (US); MUN, In K., Fremont, California 94539 (US)
(74) Representative: Müller Schupfner & Partner Patent- und Rechtsanwaltspartnerschaft mbB
(86) International application number: PCT/US2014/030171
(87) International publication number: WO 2014/145406

(56) References cited:
- WO-A1-2010/099360
- WO-A1-2012/143531
- WO-A2-2007/041375
- WO-A2-2009/042644
- US-A1- 2004 106 868
- US-A1- 2009 270 868
- US-A1- 2011 238 073
- US-B1- 7 239 908
- US-B2- 8 175 683

## Description

The invention relates generally to the field of orthopaedic surgery for the repair and replacement of damaged articular cartilage, and more specifically to a new and useful system for creating specific three-dimensional (3D) shapes in bone.

Articular cartilage is an avascular tissue that is found on the surfaces of joints and serves to provide a smooth interface upon which bones can articulate with each other. Defects in the articular cartilage can be caused by traumatic lesions or by osteochondritis dissecans or in accordance with other joint/ligament injuries. Lesions of this type in articular cartilage typically do not heal on their own, and are associated with symptoms such as joint pain, joint locking, and reduced or disturbed function. Due to the lack of vasculature in cartilage, these types of defects are known to progress to more severe forms of osteoarthritis, which may eventually require joint arthroplasty to restore joint function. Repair of these defects prior to the spreading of this articular damage may delay the need for a joint arthroplasty, especially in younger individuals. It is desirable to delay a joint replacement procedure as long as possible, especially in younger individuals, since their remaining lifespan may be longer than the lifetime of the implant. If the implant fails, the subject may require a revision procedure which is complicated and poses additional risk, inconvenience, and cost to the subject.

There are various methods that currently exist to repair cartilage. One such exemplary method is the use of osteochondral plugs. In this method, which is typically used in the knee joint, healthy osteochondral plug(s) formed of both bone and cartilage are used to replace the site of the cartilage defect. This procedure allows the chance for the cartilage surface to repair itself with healthy tissue. Larger osteochondral defects are typically filled with multiple smaller osteochondral plugs to recreate as much of the cartilage surface as possible. These osteochondral plugs may be autografts, which are taken from the same individual receiving the plug, or they may be allografts, which are taken from other individuals, living or deceased. Another conventional method that has been described is the fabrication of custom-shaped grafts for cartilage regeneration. In this procedure, an MRI scan is used to reconstruct the 3D surface of the articular cartilage including the defect. A 3D computer model is then generated to fill the cartilage defect and recreate a smooth articular cartilage surface. From this 3D computer model, a custom-shaped graft is then fabricated using synthetic or natural materials using rapid prototyping or other techniques. This custom-shaped graft is then placed into the joint to recreate a smooth surface of the articular cartilage that restores the natural surface of the joint. Another conventional method that has been described is the fabrication of synthetic scaffolds that are designed to behave mechanically like natural articular cartilage. These scaffolds are often bio-absorbable and seeded with cells that will resorb the scaffold as new cartilage matrix is created to restore healthy cartilage. These scaffolds generally consist of two components, one of which is designed to integrate with the native bone and the other that is designed to integrate with the native cartilage. Both components of the scaffold are often made of bio-absorbable or biologically active materials such that natural tissue will eventually replace them.

A common attribute of all the conventional cartilage repair techniques is that there must be a method of anchoring the cartilage replacement to the subchondral bone. There have been methods developed to remove bone plugs in the area of the cartilage defect (see U.S. Patents 5,782,835 and 6,017,348). However, these methods lack precision in locating the cartilage defect and cannot create accurate shapes that will securely fix the cartilage replacement to the bone. Additionally, it is important to minimize the amount of healthy cartilage that needs to be removed and the current methods do not provide an easy method to create a specific shape based on the individual's particular cartilage defect. Finally, it is important to achieve joint surface congruity of the articular cartilage after the cartilage replacement has been implanted to ensure normal joint contact (Koh et al. Am J Sports Med, March 2004; 32, 2 pgs. 317-320).

Furthermore WO 2007/041375 A2 and US 2011/238073 A1 disclose three dimensional printing systems that convert a computer model into a corresponding article by polymerizing or otherwise building up a shape point-by-point, which in US 2011/238073 A1 is used to form implants by a point by point 3D-printing technique.

WO 2013/033566 discloses an instrument for treating tissue during a medical procedure, the instrument including a hand-held portion and a working portion. The hand-held portion is manually supported and moved by a user and the working portion is movably coupled to the hand-held portion. A tracking device is attached to the hand-held portion for tracking the instrument. The tracking device is in communication with a control system, which is used to keep the working portion within or outside of a boundary. A plurality of actuators are operatively coupled to the working portion. The control system instructs the actuators to move the working portion relative to the hand-held portion during the medical procedure in order to maintain a desired relationship between the working portion and the boundary.

WO 2009/042644A2 discloses the features of the preamble of claim 1 and describes systems for removing cartilage and bone material wherein a robot may be used to guide the surgical and cartilage data acquisition tools.

Thus, there exists a need for a more effective process to create a precise cavity for a particular cartilage replacement implant to the subchondral bone that can allow for seamless integration of the implant into the subject's body.

A system for performing orthopaedic surgery to securely fasten an articular cartilage replacement to underlying bone is provided. The process for forming a pre-made cartilage implant intended for repair of a cartilage defect in a bone of a subject cartilage defect in a bone of a subject includes receiving scan data of the bone; creating a virtual three-dimensional model of the bone and the cartilage defect; creating a three-dimensional custom shape around the cartilage defect; and shaping a pre-made cartilage replacement implant to match the custom shape in three-dimensions. The repair of cartilage of knee joints and to other joints within the body and any other bones found within the body illustratively include the hip joint, shoulder joint, ankle joint, wrist joint, finger joint, toe joint, or other joint. The inventive orthopaedic surgery can be performed on humans or an animal of a non-human primate, a horse, a cow, a sheep, a goat, a dog, a cat, a rodent, and a bird.

The subject matter that is regarded as the invention is particularly pointed out and distinctly claimed in the claims at the conclusion of the specification. The foregoing and other objects, features, and advantages of the invention are apparent from the following detailed description taken in conjunction with the accompanying drawings in which:
FIGURE 1 is a flowchart depicting a specific embodiment of the present invention for creating a custom shaped cavity for a cartilage replacement implant based on a three-dimensional virtual model of the subject's bone and cartilage using a robotic milling system;
FIGURE 2 is a schematic block diagram of an inventive system for creating a three-dimensional model of a subject's bone and cartilage including a cartilage defect and a plan for creating a cavity that encompasses the cartilage defect;
FIGURES 3A and 3B illustrate a robotic milling tip creating a customized cavity within the articular surface of a bone, and a custom shaped cartilage implant to be placed within the cavity;
FIGURE 4 illustrates a cartilage replacement implant that includes both a cartilage type layer and a bone type layer;
FIGURE 5 illustrates a customized cartilage replacement implant, as shown in FIGURE 3B, after it has been placed within the subject's bone, as shown in FIG. 3A, resulting in a smooth surface; and
FIGURE 6 illustrates an example of a subject's bone with multiple cartilage defects in different locations

The detailed description explains the preferred embodiments of the invention.

The present invention has utility as a system for performing orthopaedic surgery to repair a cartilage defect. The following description of various embodiments of the invention is not intended to limit the invention to these specific embodiments, but rather to enable any person skilled in the art to make and use this invention through exemplary aspects thereof. Disclosed herein is a process to securely fasten an articular cartilage replacement to underlying bone. Reference will be made herein to the repair of cartilage of knee joints and it should be understood that the present invention may be applied to other joints within the body and any other bones found within the body. These other joints that are repaired through resort to the present invention illustratively include the hip joint, shoulder joint, ankle joint, wrist joint, finger joint, toe joint, or other joint. As used herein, a subject is defined as a human; or an animal of a non-human primate, a horse, a cow, a sheep, a goat, a dog, a cat, a rodent, and a bird.

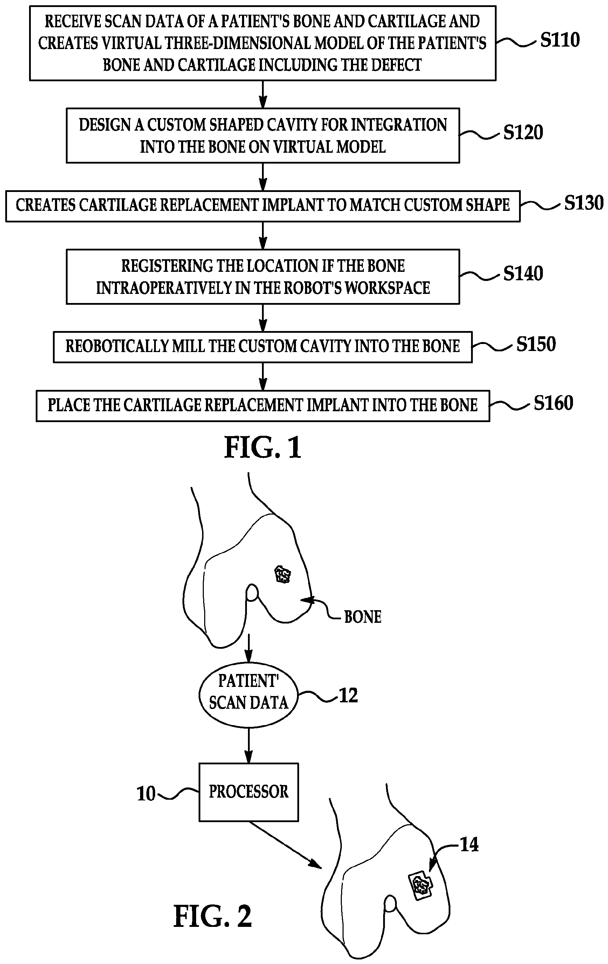

With reference to FIGURE 1, an embodiment of a process is detailed for creating a three-dimensional model of a subject's bone and cartilage and includes the steps of receiving scan data of a subject's bone and creating a virtual three-dimensional model of the subject's bone and cartilage including the cartilage defect in block S110; creating a custom shape around the cartilage defect to be removed based on the virtual model in block S120; creating or shaping a pre-made cartilage replacement implant to match the custom shape in block S130; registering the location of the actual bone during the surgery such that the precise position and orientation of the bone is known by the robot in block S140; robotically milling the custom shape into the bone in the location predetermined in the virtual model in block S150; and finally placing the graft into the bone.

Scan data of a subject's bone and cartilage for creating a virtual three-dimensional model of the subject's bone and cartilage of block S110 is readily provided from conventional sources such as CT, MRI, X-ray scans of subjects' bones, or a combination thereof. Modelling software such as VSG Amira or Medviso Segment is readily used to convert imaging scans into a model of bone of interest. The scan data may be collected by a system and process described herein or may alternatively, be collected prior to the creation of the custom milled bone shape by a systems and process specific to the bone imaging technique. It is appreciated that a physical model of the bone is readily formed with a conventional three dimensional printer or transferring the virtual model data to a computer-aided design (CAD) operated milling machine.

The design of a custom shape for integration of a cartilage replacement implant on the virtual model is performed at block S120. The custom shape in certain embodiments of the present disclosure thereby minimizes the amount of healthy cartilage margin around the defect that will need to be removed. Block S120 in other embodiments functions to create a model of the subject's bone and cartilage using surgical preoperative planning software. Additionally, block S120 in still other embodiments also functions to create instructions for a robotic system to mill out the surface of the bone to create a cavity that accurately matches the custom shape that is created. One such robotic system is the ROBODOC System, manufactured by Curexo Technology Corporation of Fremont, California.

The creation of a cartilage replacement implant to match the custom shape planned on the virtual model is provided at block S130. In some embodiments, this pre-made cartilage replacement implant is created of synthetic materials. Synthetic materials suitable for the formation of such an implant illustratively include polyglycolic acid, polylactic acid, hydrogels, agarose, and fibrin. In still other embodiments, this pre-made cartilage replacement implant is created using healthy autologous osteochondral plugs removed from a donor site within the subject, from another subject, from cadaver tissue, or tissue from another species. It is appreciated that sterilization techniques for non-autologous tissues are conventional to the art. In certain embodiments, it has been found that a cartilage replacement implant that contains both cartilage tissue and subchondral bone tissue promotes more rapid healing and implant integration (Grayson et al. 2008) due to bone tissue's vascular nature. In still other embodiments a physical model is used to evaluate the fit of the pre-made cartilage replacement implant.

The registration of the location of the bone intraoperatively within the workspace of the robot is provided at block S140. This serves to determine the precise location and orientation of the bone within the workspace of the robot. In some embodiments, this may be accomplished using fiducial markers placed into or on the subject bone or at a fixed position remote from the bone. A fiducial marker is appreciated to be a material with an opacity that is different than that of surround subject tissue such that it can be identified in an image and used as a point of reference or measure, an active device such as a radio frequency identification (RFID) tag, or a combination thereof. In still other embodiments, a registration guide is applied that fits on the bone, a surface matching algorithm, or any other method to determine the orientation of the subject bone. The usage of such techniques are further detailed in: PCT/IB2013/002311 entitled SYSTEM AND METHOD FOR REGISTRATION IN ORTHOPAEDIC APPLICATIONS. S. Cohan, "ROBODOC achieves pinless registration" The Industrial Robot; 2001; 28, 5; pg. 381. P. J. Besl, "A Method for Registration of 3-D Shapes" IEEE Transactions on Pattern Analysis and Machine intelligence, 1992; 14, pgs. 239-256.

The robotic custom shape milling into the bone is provided at block S150. In certain embodiments, the milling functions to create a matching cavity to the cartilage replacement implant to be placed within the matching cavity. It is appreciated that by milling with a slight undersize in the pre-made cartilage replacement implant, the milled custom shape placed into the bone forms a press-fit interaction with a cartilage replacement implant placed therein. It is also appreciated that any gap formed by the undersizing is readily filled with adhesive materials, bone fragment packing, bone growth promoters, or combinations thereof. Milling is readily performed with a rotary bit engaging subject bone tissue.

The placement of a cartilage replacement implant into the bone is provided at block S160. In certain embodiments, the cartilage replacement implant is formed from both a cartilage layer and bone layer such that when the implant is placed into the bone, the bone layer of the implant more rapidly integrates into the subject bone as the implant begins to heal, as compared to an implant devoid of a bone layer.

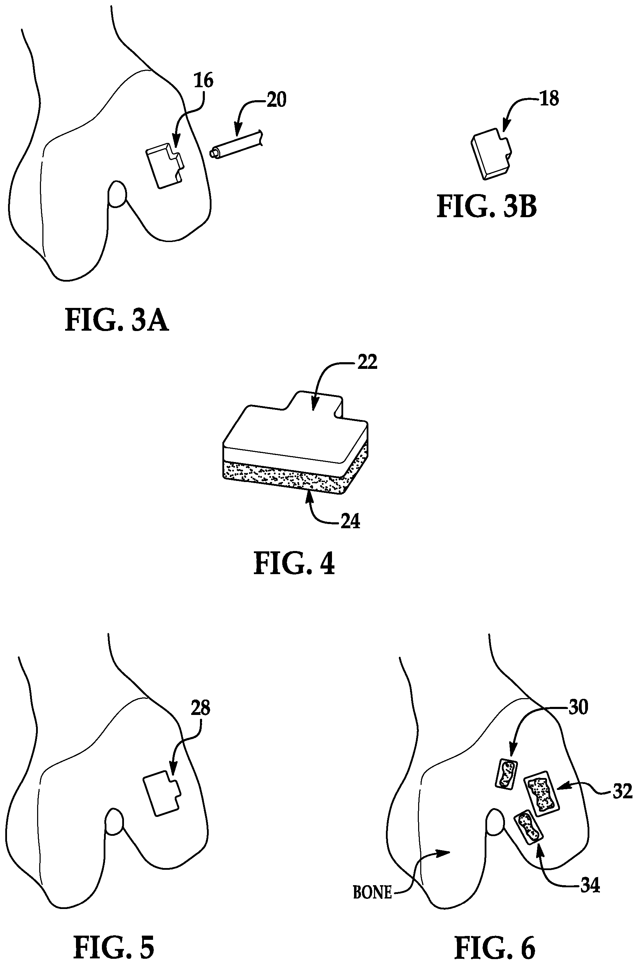

As shown in FIGURE 6, one embodiment of the system may involve a bone with multiple cartilage defects in different locations on the bone. In this embodiment, a system functions to automatically create individual customized shapes, 30, 32, and 34 for each of the defects. It is appreciated that there may be any number of individual defects so produced. The system in certain embodiments has information about the orientations of each of the cartilage defects and customized shapes with respect to each other on the bone. The robot mills custom shaped cavities 30, 32, and 34 into the bone, as shown in FIGURE 1, block S150, all automatically as the robot knows how each cavity is oriented and located with respect to each other and with respect to the bone.

As shown in FIGURE 2, a system for creating a three-dimensional model of a subject's bone of a preferred embodiment preferably includes a processor 10 configured to receive subject scan data 12 and to transform the subject scan data into a three-dimensional virtual model and allows the user to create a customized shape 14 that includes the entire cartilage defect. In some embodiments, the processor 10 may automatically create the planned customized shape 14 to include the cartilage defect and minimize the amount of healthy cartilage tissue included. The system in certain embodiments functions to create a three-dimensional model of a subject's bone based on the subject scan data input. The system functions to create a three-dimensional model of a subject's bone and cartilage in sufficient detail that the subject's real bone can be registered accurately to the three-dimensional model. The processor 10 in some embodiments automatically creates a suggested customized shape 14 that includes the cartilage defect. The processor 10 in some embodiments allows the user to modify the customized shape as they choose. The processor 10 in some embodiments creates instructions for a robotic milling tip 20, as shown in Figure 3A, to precisely mill out the cavity in the bone. The system can be used for cartilage replacement or repair for orthopaedic surgery, but may alternatively be used for any suitable applications, clinical or otherwise.

As shown in FIGURE 2, the processor 10 is configured to receive subject scan data 12 and to transform the subject scan data into a planned customized shape 14 that includes the entire cartilage defect. The processor 10 in some embodiments functions to create instructions for the creation of a three dimensional surface model based on the subject scan data. In some embodiments, the processor may run preoperative surgical planning software. In some embodiments, the scan data may be in the form of CT, MRI, or X-ray scans of subjects' bones. The scan data may be collected by the systems and methods described herein or may alternatively, be collected prior to the creation of the custom milled bone shape by systems and methods specific to imaging.

As shown in FIGURE 2, the processor 10 in some embodiments functions to create instructions for a robot to precisely mill the customized shape 14. In some embodiments, there may be multiple customized shapes 30, 32, and 34, as shown in FIGURE 6. In the case of multiple customized shapes, the processor 10 in some embodiments, functions to create instructions for a robot to precisely mill all of the customized shapes in an efficient matter based on the fact that all of the customized shapes will be in known positions relative to each other and relative to the bone.

As shown in FIGURE 3A, the robot 20 precisely mills out a cavity 16 that matches exactly with a cartilage replacement implant 18, shown in FIGURE 3B. In some embodiments, a slightly undersize the milled custom shape placed into the bone such that when the cartilage replacement implant 18 is placed into the bone, it will fit tightly in a press-fit.

As shown in FIGURE 4, the cartilage replacement implant includes a layer of cartilage 24 and a layer of bone 26. In some embodiments the layer of cartilage 24 and layer of bone 22 may be synthetic or natural. In some embodiments, the cartilage replacement implant will only have a layer of cartilage and not include a layer of bone.

As shown in FIGURE 5, the cartilage replacement implant 18 is placed into the cavity 16 such that it creates a smooth surface 28 that matches with the healthy cartilage on the bone. A layer of bone 26, as shown in FIGURE 4, will be of the same thickness as the removed native bone and the layer of cartilage 24, shown in FIGURE 4, will be of the same thickness as the surrounding cartilage.

In some embodiments of the system, there may be a reason during the surgical procedure or during another surgical procedure to return to the precise location where the customized shape has been created. If the bone location is still registered or re-registered within the workspace of the robot, the robot will be able to precisely guide its tip to the location of the customized shape for an additional procedure or any other reason.

As a person skilled in the art will recognize from the previous detailed description and from the figures and claims, modifications and changes can be made to the preferred embodiments of the invention without departing from the scope of this invention defined in the following claims.

## Claims

1. A system for performing orthopaedic surgery to repair a cartilage defect of a subject's bone,
comprising a processor (10), configured to receive subject scan data (12) of the cartilage defect and to transform the subject scan data (12) into a three-dimensional virtual model of the bone and the cartilage defect with registration of the location of the bone intraoperatively within the workspace of a robot (20) to determine the precise location and orientation of the bone within the workspace of the robot, and comprising a robotic milling tip (20),
wherein the processor (10) is capable of creating at least one customized shape (30,32,34) around the cartilage defect,
**characterized in that**
the processor (10) is capable of creating instructions for the robotic milling tip (20) to mill out the at least one customized shape (30, 32, 34) in the bone that matches with a cartilage replacement implant (18).

2. The system of claim 1,
comprising fiducial markers adapted to be placed into the bone, onto the bone or at a fixed position remote from the bone of the subject.

3. The system of claims 1 or 2,
comprising a computer-aided design (CAD) operated milling machine to form a physical model of the bone.

4. The system of any of claims 1 to 3,
wherein the robotic milling tip (20) comprises a rotary bit adapted to engage the subject's bone tissue.

5. The system of any of claims 1 to 4,
wherein the processor (10) creates instructions for the robotic milling tip (20) to mill the customized shape slightly undersized into the bone to form a press-fit with a pre-made cartilage replacement implant when placed therein.

## Patentansprüche

1. System zur Durchführung von orthopädischen Operationen zur Reparatur eines Knorpeldefektes im Knochen eines Patienten,
aufweisend einen Prozessor (10), der so konfiguriert ist, dass er Patienten-Scandaten (12) des Knorpeldefekts empfängt und die Patienten-Scandaten (12) in ein dreidimensionales virtuelles Modell des Knochens und des Knorpeldefekts unter Registrierung der Position des Knochens intraoperativ innerhalb des Arbeitsraums eines Roboters (20) transformiert, um die genaue Position und Orientierung des Knochens innerhalb des Arbeitsraums des Roboters zu bestimmen, und
aufweisend eine Roboter-Frässpitze (20),
wobei der Prozessor (10) in der Lage ist, mindestens eine angepasste Form (30, 32, 34) um den Knorpeldefekt herum zu erzeugen, **dadurch gekennzeichnet, dass** der Prozessor (10) in der Lage ist, Anweisungen für die Roboter-Frässpitze (20) zu erstellen, um die zumindest eine angepasste Form (30, 32, 34) aus dem Knochen herauszufräsen, die mit einem Knorpelersatzimplantat (18) übereinstimmt.

2. System gemäß Anspruch 1,
aufweisend Referenzmarkierungen, die in den Knochen, auf den Knochen oder an einer ortsfesten, vom Knochen des Patienten entfernten Stelle angebracht werden können.

3. System gemäß Anspruch 1 oder 2,
aufweisend eine mit computerunterstütztem Design (CAD) gesteuerte Fräsmaschine zur Erstellung eines physikalischen Modells des Knochens.

4. System gemäß einem der Ansprüche 1 bis 3,
wobei die Roboter-Frässpitze (20) einen rotierenden Meißel umfasst, der geeignet ist, in das Knochengewebe des Patienten einzugreifen.

5. System gemäß einem der Ansprüche 1 bis 4,
wobei der Prozessor (10) Anweisungen für die Roboter-Frässpitze (20) erstellt, um die angepasste Form mit leichtem Untermaß in den Knochen zu fräsen, um eine Presspassung mit einem vorgefertigten Knorpelersatzimplantat zu bilden, wenn es darin eingesetzt wird.

## Revendications

1. Système pour exécuter une opération orthopédique destinée à réparer un défaut de cartilage dans un os d'un patient,
comprenant un processeur (10), configuré pour recevoir les données de scannage du patient (12) concernant le défaut de cartilage et pour transformer les données de scannage du patient (12) en un modèle virtuel tridimensionnel de l'os et du défaut de cartilage avec une concordance de l'emplacement de l'os de manière intra-opératoire à l'intérieur de l'espace de travail d'un robot (20) pour déterminer avec précision l'emplacement et l'orientation de l'os à l'intérieur de l'espace de travail du robot, et comprenant une pointe de fraisage robotisée (20),
dans lequel le processeur (10) est capable de créer au moins une forme personnalisée (30, 32, 34) autour du défaut de cartilage,
**caractérisé en ce que**
le processeur (10) est capable de créer des instructions pour la pointe de fraisage robotisée (20) afin de fraiser ladite au moins une forme personnalisée (30, 32, 34) dans l'os qui s'apparie à un implant de remplacement de cartilage (18).

2. Système selon la revendication 1,
comprenant des marqueurs de référence adaptés pour être placés dans l'os, sur l'os ou à une position fixe éloignée de l'os du patient.

3. Système selon la revendication 1 ou 2,
comprenant une machine de fraisage dotée d'une conception assistée par ordinateur (CAO) pour former un modèle physique de l'os.

4. Système selon l'une quelconque des revendications 1 à 3,
dans lequel la pointe de fraisage robotisée (20) comprend un embout rotatif adapté pour engager le tissu osseux du patient.

5. Système selon l'une quelconque des revendications 1 à 4,
dans lequel le processeur (10) crée des instructions pour la pointe de fraisage robotisée (20) afin de fraiser dans l'os la forme personnalisée d'une taille légèrement inférieure pour former un emmanchement serré avec un implant de remplacement de cartilage réalisé au préalable quand celui-ci est placé à l'intérieur.
